# EUROPEAN PATENT SPECIFICATION

(11) **EP 0 526 226 B1**
(45) Date of publication and mention of the grant of the patent: **28.05.1997**
(21) Application number: 92306986.8
(22) Date of filing: 31.07.1992
(51) Int. Cl.: G01N 33/52, C12Q 1/54, C12Q 1/60

(54) **Device and method for conducting biological assays containing particulate screening system**
Vorrichtung und Verfahren zur Durchführung biologischer Assays, die ein Partikelsiebsystem enthalten
Procédé et dispositif pour exécuter des essais biologiques contenant un système pour tamiser des particules

(30) Priority: 02.08.1991 US 739639
(43) Date of publication of application: 03.02.1993
(73) Proprietor: ORTHO PHARMACEUTICAL CORPORATION, Raritan New Jersey 08869-0602 (US)
(72) Inventor: Poto, Edward M., Hillsborough, New Jersey 08876 (US); Costaris, Arthur W., Jutland, New Jersey 08827 (US); Armington, Susan Reeves, Trenton, New Jersey 08618 (US); Santiago, Manuel F., Howell, New Jersey 07731 (US)
(74) Representative: Mercer, Christopher Paul

(56) References cited:
- EP-A- 0 045 476
- EP-A- 0 287 731
- EP-A- 0 295 526
- EP-A- 0 392 377
- US-A- 3 552 928
- US-A- 4 361 648

## Description

### Background of the Invention

The present invention relates to an analyte test based upon the use of dry reagent chemistry. Dry reagent chemistry is a diagnostic area distinct from more classical wet reagent chemistry. Wet reagent chemistry requires that the reagent chemicals be stored in separate solution containers before use. These chemicals are then brought together into a common reaction chamber during the test procedure. The process of reconstituting and mixing the chemicals generally requires automation or manual intervention by the test operator.

By contrast, in dry reagent chemistry, the reagent chemicals are stored in a dry state in a single device which also incorporates a reaction chamber as part of the structure. This integration of the different components has the advantage of requiring much less automation or operator intervention. Fluid contained in the sample activates the test chemicals, and the matrix that holds the test chemicals usually undergoes a color change that can be read directly without further processing. Such devices are often referred to as "dipsticks" or "test strips".

Many such test strips have been developed for the rapid analysis of body fluids for various constituents whose presence and/or quantity may relate to a pathological condition.

The state of the art for dry reagent based biological fluid testing typically involves the use of complex, multi-layered, composite structures. For example, in testing whole blood samples, the requisite composite structures generally contain one or more sections to separate out plasma from whole blood cells, sections to facilitate sample spreading, and one or more sections which contain the analyte detection chemistry.

U.S. patent #4,810,470 to Miles describes a complex diagnostic device for the quantitative determination of analyte concentrations in liquid samples. Particular analytes mentioned include creatinine, cholesterol, triglycerides, potassium, and the like. The device includes one or more test reagent-treated bibulous matrices covered at least partially by an impermeable coating or film. The liquid sample is metered into the bibulous matrix by this impermeable coating.

One particular analyte has been the subject of increasing interest, and that is cholesterol. This is especially so in recent times, due to the public awareness of the health risks associated with continuous high cholesterol blood levels. The basic chemistry involved in testing blood cholesterol levels is well-known. U.S. Patent No. 3,907,645 (Richmond) discloses a method and kit for assaying cholesterol in a liquid. The disclosed cholesterol assay involves incubating the liquid to be tested with an enzyme preparation which is derived from *Norcardia* species. The enzyme preparation oxidizes any cholesterol present in the liquid into cholestenone and hydrogen peroxide. The amount of cholesterol is determined by measuring the amount of hydrogen peroxide produced.

U.S. Patent No. 3,925,164 (Beauchamp et al.) discloses a method and reagent composition for determining cholesterol in a sample. In this method, a sample is treated with cholesterol esterase obtained from a microorganism, thereby releasing any bound cholesterol in the sample. The resulting cholesterol content of the sample is then determined using standard methods. The reagent composition comprises cholesterol esterase obtained from a microorganism (e.g., *Candida* sp., *Rhizopus* sp., *Aspergillus* sp. ) and a system for the determination of free cholesterol. The composition of cholesterol esterase, cholesterol oxidase, catalase, acetyl acetone, methanol, and a buffer containing ammonium ions is disclosed.

U.S. Patent No. 4,212,938 (Gruber et al.) teaches a test composition and method for determining total cholesterol in a body fluid sample comprising a chemical system having cholesterol ester hydrolase activity and a means for determining at least one of the reaction byproducts. A test composition of cholesterol oxidase, cholesterol esterase, and a system for determining hydrogen peroxide (i.e. peroxidase, a chromogen, and a buffer) is disclosed. For example, U.S. Patent No. 4,186,251 (Tarbutton) discloses a test device for use in determining total cholesterol in a biological fluid sample. The device includes a test composition and a carrier for the test composition. The test composition includes a chemical system having cholesterol ester hydrolase activity, a chemical system having cholesterol oxidase activity, and a means for determining hydrogen peroxidative activity (i.e. a colorimetric response).

The basic test systems work on the basis of a wet-chemistry system. However, it should be noted that it is critical in any blood chemistry testing system that the red blood cells be separated from the blood plasma. Red blood cells interfere with the colorimetric end result, due to leakage of their hemoglobin and obstruction of proper colorimetric analysis. In the wet chemistry systems, the red blood cells may be separated out prior to testing. This separation may also be done prior to application of the sample into the dry chemistry test systems. However, certain disclosed dry chemistry systems have elaborate means of effecting this separation within the test system itself. U.S. Patent No. 4,753,776 (Hillman et al.) discloses a method and device for separating plasma from red blood cells. A capillary flow device having a low pressure glass fiber filter composed of borosilicate glass fibers is used to effect the separation.

U.S. Patent No. 4,604,264 (Rothe et al.) discloses a multi-layer test strip. The test strip comprises a carrier layer (multifilar fabric or fleece); a dry, reagent-containing film layer; and a synthetic resin film layer. In a marketed product based upon this invention, a multi-layered strip contains a number of blood cell separation layers. The cholesterol detection chemistry is coated on the underside of a transparent plastic support. In use, the user places a measured amount of whole blood on a screened pad. Blood soaks through into a second glass fiber pad below. The glass fiber pad separates plasma from whole blood. Plasma then diffuses to an area below the cholesterol detection layer. The cholesterol detection layer is mechanically pressed into the plasma containing layer. The plasma activates the detection chemistry, resulting in a color change. This color change is then read through the plastic support layer by a photometer, and a microprocessor is used to compute the equivalent concentration of cholesterol.

U.S. Patent No. 3,630,957 (Rey et al.) discloses a diagnostic test strip. The test strip comprises a homogeneous water-resistent film having a color forming reagent dispersed throughout. The device may also include a carrier support for the film. A number of film compositions are disclosed including polymers, cellulose, glass, and synthetic resins.

U.S. Patent No. 3,552,928 (Fetter et al.) discloses a test device for detecting soluble constituents in whole blood. The device has two bibulous matrices, which may be laminated to each other. One matrix contains the test reagent and the other an amino acid separating reagent.

U.S. Patent No. 4,477,575 (Vogel et al.) discloses a process and device for separating plasma or serum from whole blood using a layer of glass fibers.

German Patent 2512586 concerns a dry reagent based cholesterol test in which red cells are separated from plasma before the test starts. A fixed volume of plasma is applied to a multi-layered slide that looks very much like a 35mm photographic slide. This slide contains a sample spreading layer, and one or more layers containing the cholesterol detection chemistry. The reaction causes a color change to occur on the underside of the slide. After incubation at 37°C for several minutes, the color is then read from below by a photometer device and the equivalent cholesterol concentration determined by a microprocessor.

The above-mentioned dry chemistry systems represent some of the most notable the state-of-the-art in multi-lamellar devices for the measurement of glucose, cholesterol, and other such analytes in biological fluids. However, there is a need for a simple, easy to use device that will allow rapid, accurate, colorimetric testing of very small quantities of a biological sample of about twenty microliters or less, without interfering leakage of particulates such as red blood cells (or the product of their lysis - hemoglobin) into the colorimetric detection area. Such a device would be suitable for doctor's office applications, or even home use for those people wishing to monitor the level of certain components in their blood, urine, saliva, cerebrospinal fluid, or other bodily fluid or biological sample suspended in liquid form. Typical analytes include glucose, cholesterol, uric acid, ascorbic acid, bilirubin, carotene, hormones, protein, certain enzymes, alcohol, toxins, drugs and the like. Such a device would be particularly useful to those persons suffering from unacceptable blood glucose or cholesterol levels that they are trying to correct through diet, exercise, and other means of treatment, including drug therapy.

### Summary of the Invention

The invention relates to a device and method for the determination of an analyte in a biological fluid comprising an initial hydrophilic screening layer, at least one final hydrophilic screening layer, and a reagent membrane pad with at least one hydrophilic microporous membrane having incorporated within it a colorimetric detection system for such analyte. The device and method of the invention are adapted to detect, and in some cases quantitatively or semi-quantitatively measure, an analyte present in a biological fluid so that a color change is observed when said fluid is brought into contact with the reagent membrane pad containing the detection system. The system is particularly suited in glucose or cholesterol detection devices where presence of particulate matter in the biological sample, most notably red blood cells, would be a hindrance to the analytical results.

### Brief Description of the Drawings

Figure 1(a) is a test strip in accordance with the invention containing a glycine paper initial particulate screening layer and two polycarbonate final screening layers.

Figure 1(b) is a similar test strip having only one polycarbonate final screening layer.

Figure 2 is a schematic of a hand-held color chart to compare the reaction site color to standardized colors indicative of various levels of analyte.

### Description of the Invention

The analyte test strip of the present invention represents a simple, accurate approach to dry reagent testing. The device of the invention comprises a system of hydrophilic particulate screening layers in conjunction with a reagent membrane pad incorporated with a chemical detection system for the desired analyte. As used herein, "hydrophilic" refers to those compositions that by their very nature have a strong affinity for water, or have been coated or in some other manner treated so that they have a strong affinity for water. The various screening layers in the device as claimed herein are hydrophilic to ensure appropriate wettability when contacted with blood or other biological fluids, so that such fluid will flow through each layer to make contact with the next layer proximate thereto.

The screening layer system of the device claimed herein comprises an initial hydrophilic screening layer that serves to remove a substantial portion of particulates from the biological sample, such as red blood cells from the plasma portion of a blood sample, contaminating red blood cells in biological samples other than plasma, other interfering particulate substances, such as cellular debris in urine or saliva, and the like. Suitable materials for this layer of the device are those that will trap a substantial portion of the interfering substances, without disrupting them and possibly releasing undesired components (such as hemoglobin in the case of interfering red blood cells). For example, the preferred initial screening layer will trap a substantial portion of red blood cells in a manner that is gentle enough so as to avoid disrupting their membranes and allowing their hemoglobin to leak. Suitable membranes for this purpose are bibulous membranes such as those made out of cellulose, polysulfone, polyester, glass fibers and the like, all hydrophilic in their nature or treated chemically or physically to render them hydrophilic by commonly accepted methods. Exemplary methods include treatment with surfactants such as detergents to modify the surface tension, or changing the charge of the materials by chemical or physical means, irradiation, and the like. Particularly preferred for use herein is paper treated with glycine or serine, such as disclosed in U.S. patent #3,552,928.

Preferred thicknesses of such initial screening layer range from about .30 mm to about 1mm. The thinner the first layer, the less void volume to retain the biological sample, and the less sample required to carry out the test. This becomes quite important in home use of the presently claimed device when testing blood, as the user must obtain the blood sample from a fingerprick. Thus, it can be appreciated that it is desirable to minimize the size or number of such fingerpricks, to maximize the comfort of the user. Additionally, in the preferred embodiments, the device of the invention is constructed using principles of "island placement", ie: the area to which the biological sample is applied forms a small island in the middle of the strip itself. Here again, this minimizes the amount of biological sample required and avoids spillage of the sample (particularly when that sample is blood) onto the surrounding area of the strip or even onto the instrument area in those situations wherein the strip is placed into a meter for reading of the colorimetric results.

The overall dimensions of this first layer may be determined by those skilled in the art in accordance with the intended size and design of the resulting test strip, and keeping in mind that the larger the first layer, the more void volume to retain the sample.

The device of the invention further comprises at least one, final screening layer to catch particulates that may have leaked through the initial screening layer. This second layer also serves to regulate the sample flow through the device. In this regard, it should be noted that the reagent membrane pad is typically dry and absorbent, and thus, tends to pull the biological sample through the device once fluid begins to contact this area. A delicate balance needs to be maintained at this point to avoid a flow of fluid so strong that the contaminating particulates are sheared in the process. For example, in a sample containing red blood cells, a strong flow of the plasma or other sample medium could result in shearing of the red blood cells, with concomitant release of the undesirable contaminating hemoglobin. Accordingly, suitable membranes for this purpose are those that will allow the fluid to flow through at a somewhat steady state, but will trap the interfering particulates. Of these may be mentioned polycarbonate, polyester, polysulfonate, nylon, and the like membranes, or those comprising combinations of such materials.

In the preferred embodiments, the presently claimed test strip is used to test whole blood for analytes of interest. Preferred for use herein as the final screening layer or layers in such test strip are polycarbonate membranes having pores small enough that the red blood cells cannot squeeze through, and typically about .2 micrometers to about 3 micrometers. Suitable thicknesses of each layer are those thicknesses that will not affect the void volume of the material to such an extent that it would tend to substantially prevent the sample fluid from reaching the intended reagent membrane pad. Generally speaking, about two times the diameter of a red blood cell is suitable for this purpose, and particularly about 12 to about 14 micrometers. Suitable numbers of pores are those that serve to optimize the monitoring of the fluid flow so that it is not too fast, increasing the chances that the red cells will burst from the force. Particularly preferred numbers of pores range from about 2x10⁶ - 1x10⁸ per square centimeter, most preferred being about 2x10⁷ - 3x10⁷ per square centimeter. If more than one final screening layer is employed, membrane material having fewer pores that are generally larger in diameter is chosen. For example, if two layers are used, membrane having approximately 2x10⁷ pores per square centimeter, with a pore diameter of approximately 1 micrometer is preferably used, compared to the single final screening layer construct, wherein a membrane having approximately 3x10⁷ pores per square centimeter, with a pore size of approximately .6 micrometers is preferred. Finally, as in the case of the initial screening layer, overall dimensions may be optimized by those skilled in the art, to make appropriate contact with the other layers in the device, in accordance with the overall size and design.

The test strip further comprises a reagent membrane pad, which is comprised of one or more bibulous carrier matrices generally known to the art for the support of all or a portion of a dry chemistry analyte detection system. The carrier matrix or matrices may be comprised of nylon, filter paper, polysulfone, polyvinylidene difluoride, polyester, cellulose, glass fiber, and the like materials suitable for such purpose. It is generally preferred that positively charged nylon be employed in this capacity and that the entire dry reagent analyte detection system, including the chromogenic portion of that detection system, be incorporated into one membrane, if possible.

The reagent membrane matrix or matrices are impregnated with a suitable dry chemistry analyte detection system, in accordance with the particular analyte to be detected. Typical analytes are glucose and other sugars such as galactose in urine and blood, protein in urine, alcohol in saliva or blood, ketone bodies, carotene, creatinine, lactose, pentose, lactic acid, ascorbic acid, hippuric acid, pyruvic acid, porphyrin, cystine, and other amino acids, melanin, nucleic acids, phospholipids, urea nitrogen, uric acid, phenylalanine, free fatty acids, monocarbohydrates, cholinesterase, amylase, and certain other enzymes, hormones, drug substances, and toxins, infectious disease analytes such as HIV and hepatitis (typically using immunoassay colorimetric techniques such as biotin-avidin, enzyme labels, colloidal gold labels, and the like) only to mention a few. Known dry chemistry reagent systems for detecting analytes such as those just mentioned are too numerous to present in their entirety here. However, specialty chemicals, such as diagnostic reagent grade enzymes are widely available on a commercial basis. One such company is Toyobo Co., Ltd. of Osaka, Japan, whose catalog also conveniently presents chemical reaction schemes for various analytes, adaptable for the most part to the dry chemistry analyte detection systems useful in the test strip presented herein. A few representative patents also describing useful reagent systems are U.S. patent #4,935,346 to Lifescan, Inc. U.S. patent #4,478,942 to Fuji Photo Film Co., Ltd., U.S. patent #4,710,458 to R.J. Harvey Instrument Corporation describing a urease method for determination of blood urea(Col. 9), and U. S. patents #4,273,868 and 4,361,648 to Miles Laboratories, Inc.

The test strip described herein is particularly suited for the detection and/or quantitation of cholesterol. Cholesterol is generally present in blood serum or plasma as part of many different protein-lipid complexes. For example, high density lipoproteins and low density lipoproteins have differing amounts of cholesterol, phospholipids, protein, triglycerides, and the like. These particles are broken down to yield cholesterol esters which may then be quantitated in accordance with the invention. Accordingly, in one preferred embodiment the reagent membrane pad of the invention, comprising a single matrix, is impregnated with a dry chemical cholesterol detection system comprising a component to solubilize the lipid particles and produce cholesterol esters, a component that hydrolyzes the fatty acid from the cholesterol ester yielding free cholesterol, and a component that oxidizes the cholesterol yielding hydrogen peroxide as a by-product whose formation may be measured colorimetrically as a function of the presence of cholesterol. Certain exemplary cholesterol detection chemistries may be found, inter alia, in U.S. patent numbers #4,008,127; 3,925,164; 4,503,144; 4,144,129; 4,212,938; and 4,186,251. Suitable components for solubilization of the cholesterol particles are detergents such as sodium cholate, 3-[(3-cholamidopropyl)-dimethyl-ammonio]-1-propanesulfate (CHAPS), octylphenoxypolyethoxyethanol, hydrogenated (Triton X-100™ available from Sigma Chemical, Missouri) deoxycholate, and the like. Suitable components for hydrolyzing the cholesterol esters are enzymes such as cholesterol esterase or nonspecific esterases capable of hydrolyzing cholesterol esters, such as pancreatic or microbial cholesterol esterase, and microbial lipoprotein lipase. Suitable oxidases are enzymes or precursors having cholesterol oxidase activity and capable of oxidizing the cholesterol into cholestone with the liberation of free hydrogen peroxide, such as cholesterol oxidase, which liberation may be measured colorimetrically.

In the preferred embodiments, the dry chemistry reagent membrane pad of the test strip contains a colorimetric (chromogenic) detection system for expression of the end results, preferred being those systems based on release of hydrogen peroxide. It is preferable that this detection system is incorporated in the same matrix as the previously mentioned dry chemistry analyte detection systems, but in some cases may be incorporated into its own matrix adjacent to the analyte detection matrix.

A preferred chromogenic means for determination of released hydrogen peroxide is the use of a substance have peroxidative activity and an oxidation-reduction indicator such as: AAP/HBSA (4-aminoantipyrine/4-hydroxy-benzenesulfonic acid), AAP/PDP (primaquine diphosphate), MBTH/DMAB (3-methyl-2-benzothiazolinone hydrazone/dimethyl amino benzoic acid), Viomedics Indophane Substrates, 3,3¹,5,5¹-tetraalkylbenzidine compounds as disclosed in U.S. patent 4,385,114 and specifically TMB (3,5,3,5-tetramethylbenzidine), m-anisidine compounds such as those disclosed in U.S. patent #4,391,905, o-dianisidine, o-toluidine, o-tolidine, benzidine, 2,2'-azinodi-(3-ethylbenz-thiazoline sulphonic acid), 3-methyl-2-benzothiazoli-none hydrazone plus N,N-dimethylaniline, phenol plus 4-aminophenazone, sulfonated 2,4-dichloro-phenyl plus 4-amino-phenazone, 3-methyl-2-benzothiazoli-none hydazone plus 3-(dimethylamino)benzoic acid, 2-methoxy-4-allyl phenol, 4-Aminoantipyrine-dimethylaniline, 0-phenyl diamine (OPD), and the like. Certain of the patents cited above may also describe these or other colorimetric systems.

H is further preferred that the test strip comprises a chemical agent incorporated into said reagent membrane to prevent excessive wash-out of said chromogenic detection system. Preferably, the chemical agent comprises a high molecular weight polymer such as dextran.

The reagents may be applied to the reagent membrane pad matrix or matrices in any suitable manner, including dipping, spraying, and the like. In the preferred embodiments, the reagents are added sequentially in two additions, as they require different solvents. The reagent test strips comprising the various layers as described herein may be assembled so that the initial screening layer comes into contact with the biological sample first, the final screening layer or layers second, and the reagent pad last. In the preferred embodiments, each one of the layers described is in intimate contact with the layers proximate to it. Air bubbles should be avoided in the construction of the devices, as they prevent those particular portions of the device from filtering properly. The device may also contain sample receiving areas, handles, suitable supports or housings for the layers, carriers and the like, all in accordance with its intended applications. Supports, housings, and carriers are generally made from inert materials chosen from various sources including certain plastics, papers, glass, Teflon™, metals, wood, and the like. The test strips and layers themselves may be of varying dimensions, depending again on intended use.

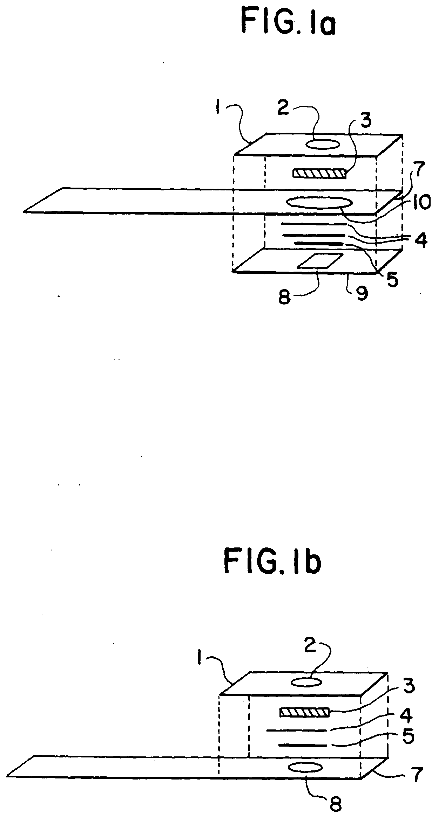

By way of example of a suitable assembly, reference is made to Figure 1(a) and (b). Tape 1 defining a blood sample inlet aperture 2 is positioned over glycine paper 3 so that aperture 2 is centered on top of paper 3. Polycarbonate layer 4 is centered under this assembly, with reagent pad 5 positioned underneath. This multilamellar structure is affixed to plastic handle 7 having an aperture 10 in 1(a) or 8 in 1(b). In Figure 1(a) there is also a bottom tape 9 with a reading aperture 8. Reading aperture 8 is positioned so that it aligns with aperture 2 (and 10 where applicable). The colorimetric response is read from the bottom through aperture 8, either visually by turning the device over to look at the results and possibly compare with a color chart, or by contacting the test strip with an instrument capable of electronically detecting the result. One skilled in the art will appreciate that suitable instruments useful for this purpose might be similar to those commercially available such as OneTouch II™ available from Lifescan Inc., the Tracer II®, Accu-Chek® II™, Accu-Chek® Easy™, Accu-Chek® II Freedom™, and the Reflotron® from Boehringer Mannheim Corporation, Clinitek® 100 System and Glucometer™, available from Ames Division of Miles Laboratories, Rapidimat® II/T available from Behring Diagnostics Inc., Companion® Z available from Medisense, Inc., and the Answer™ product available from Wampole Laboratories. Other suitable instruments are similar to those described in U.S.patent #4,935,346.

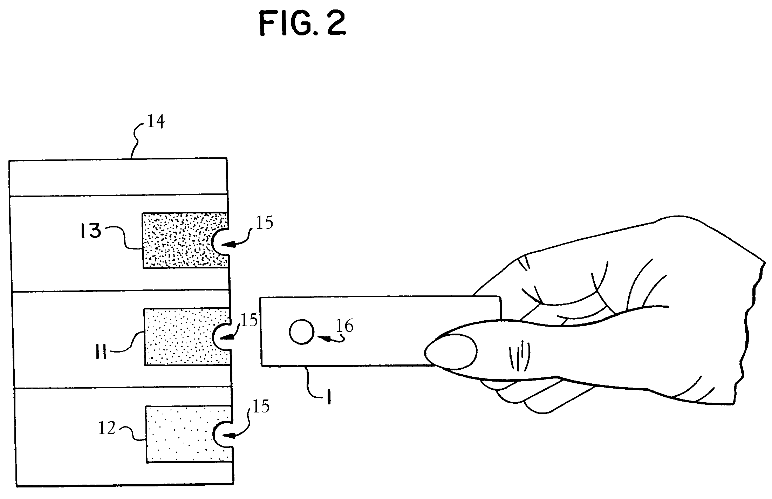

The reagent strips as described herein may also be used as a handheld color chart reagent test such as that shown in Figure 2. The user obtains a reaction color where the colorimetric reaction has taken place through a cholesterol detection chemistry system such as that just described. The strip 1 is placed behind the color chart 14 so that the strip color in the reactive zone 16 is next to a notched area 15 of the color chart 14. If the strip color matches the desirable color 12 or is between desirable and borderline 11 colors, the blood cholesterol is less than 200 mg/dl. If the strip color matches the borderline color 11 or is between borderline and high color 13, the blood cholesterol level is in the borderline range of 200 mg/dl to 239 mg/dl. If the strip color matches the high color 13 or is even a darker color, such as a darker blue if the TMB system is used, the blood cholesterol is in the high range, which is greater than or equal to 240 mg/dl. These ranges are established by the U.S. Department of Health and Human Services of the National Institutes of Health, National Cholesterol Education Program (Report of the Expert Panel).

The following examples depict more specific embodiments of the present invention, but should not be considered limitative thereof.

### EXAMPLE 1.

### Cholesterol test strips

Dry chemistry reagents may be coated on a number of different membranes such as nylon, polysulfone, polyvinylidene difluoride, polyester, cellulose or glass fibre. Preferentially the membrane is a positively charged nylon such as:
Biodyne B, 0.8 um from Pall Biosupport (Glen Cove, NY), Zetabind, 0.8 um from Cuno Inc. (Meriden, Connecticut) or a Magna Nylon from Micron Separation, Inc. (Westborough, MA).

The above-mentioned membranes were coated with reagents in a two step process. In the first step the membrane was immersed into an organic solvent based solution containing:
824 ml of dimethyl sulfoxide,
12.02 g 3,3',5,5'-tetramethylbenzidine,
100 ml of 50 % (w/v) dioctyl sulfosuccinate, sodium salt in methanol,
75.8 ml of 0.66 M sodium cholate in water.

The membranes were then dried at 56 °C for 15 to 20 minutes. To avoid damage to certain of these membranes, the solvent, dimethyl sulfoxide, was replaced or reduced in concentration by methanol. In such cases, the equivalent amount of 3,3',5,5'-tetramethylbenzidine was added as a 0.2 M solution in acetonitrile or as a 2 M solution in dimethyl sulfoxide.

In a second step, the dry membrane was coated in an aqueous solution containing reagents added in the following sequence with thorough mixing between each addition:
20g, dextran mol.wt. 5,000,000 - 40,000,000,
43g sucrose,
5g Gantrez AN-139 Polymer (GAF Chemical Co.),
50 ml of 1 M phosphate, pH 7.2 (0.745 moles Na₂HPO₄, 0.255 moles KH₂PO₄),
10g polyvinyl-pyrrolidone, mol. wt. 40,000, and
25,000 units of cholesterol oxidase, 150,000 units of horseradish peroxidase and 295,000 cholesterol esterase units of lipoprotein lipase to a final volume of 11.

The coated membrane was dried for 15 to 20 minutes at 56 °C.

The test strip (and as illustrated in Figure 1b) was assembled by placing a 6 mm x 6 mm square reagent membrane over the reading hole onto the adhesive side of the polystyrene plastic handle. The reading hole was cut through both the polystyrene plastic handle, and adhesive (such as 3M A415). The next layer is a 12.7 mm x 19 mm polycarbonate or polyester membrane with the 0.6 um pore size and 3 x 10⁷ pores/cm² (such as Nucleopore's 0.6 um polycarbonate or polyester membrane). Polycarbonate membrane overlaps the reagent membrane and binds to the adhesive underneath serving as the final barrier to red cells. This membrane was also coated with a 2 g/l of Triton X-100 in water and dried. The blood separation filter is the next layer which is covered by a 19 mm x 19 mm single side adhesive tape so that blood application hole on the adhesive tape is directly above the reading hole on the plastic handle beneath. The blood separation filter is a 5 mm x 5 mm square paper such as Sigma's medium thickness blotting paper or Whatman™ 31 ET Chrom paper coated with the solution containing: 250 g/l glycine, 5.5 g/l NaCI and 2.978 g/l disodium ethylenediamine-tetraacetate (EDTA). The paper is then dried for 15-20 min at 56 °C.

The cholesterol test strip as assembled above can be used as a semi-quantitative visual test or a semi-quantitative or quantitative meter test. For the meter-based test, the strip is inserted into a meter receiving port with the blood receiving side facing up. When prompted by the meter a drop of blood (about 20 ul) is applied to the strip. The meter gives results as soon as the maximum color is detected, which is usually well within two minutes. For the semi-quantitative visual test, a drop of blood is applied to the strip. After two minutes the color on the reading side of the test strip is compared to the color chart.

### EXAMPLE 2

### Cholesterol test strips with two polycarbonate membranes

The test strip was constructed as described in Example 1 but instead of using the single polycarbonate or polyester membrane, two polycarbonate or polyester membranes with 1.0 um pore size and pore density of 2 x 10⁷ pores/cm² were used. This test strip is illustrated in Figure 1a. Membranes were also coated with a solution made from 2 g/l of Triton X-100 in water and dried. To improve binding of strip layers, the first polycarbonate membrane is smaller (10 mm x 10 mm) and it is placed above the reagent membrane using the island placing technique. The second polycarbonate membrane, 12.7 mm x 19 mm, overlays the first polycarbonate membrane binding to the adhesive on the plastic handle below as well as to the adhesive tape on the blood application above. The larger polycarbonate membrane also overlays the blood separation filter which is now between the larger polycarbonate membrane and the adhesive tape on the blood application side.

Polycarbonate and polyester membranes used have ranged in pore sizes from 0.2 to 3 um and in pore density from 2 x 10⁶ to 3 x 10⁸ pores/cm². Preferably use one polycarbonate or polyester membrane with 0.6 um pore size and 3 x 10⁷ pores/cm² or two polycarbonate or polyester membranes with the 0.8 to 1 um pore size and 2 x 10⁷ to 3 x 10⁷ pores/cm².

### EXAMPLE 3.

### Alternate blood separation filters

In further experimentation, the initial blood separation filter described in the Example 1 was substituted with the following filters with good results,
LK9 or LK6 membranes developed by Pall Biosupport (Glen Cove, NY) treated with a solution of 20 % glycine (w/v) and 0.05 % SDS (w/v) in water and dried.
Cellulose paper, such as Sigma's medium thickness blotting paper, Whatman™ 31 ET Chrom paper, Schleicher & Schuell 903, 598 or 740-E analytical paper, coated with the solution containing: 50 g/l glycine, 50 g/l serine and 4.4 g/l NaCI. Paper was dried for 15-20 min at 56 °C.
Cellulose paper, such as Sigma's medium thickness blotting paper, Whatman™ 31 ET Chrom paper, Schleicher & Schuell 903 or 740-E analytical paper, coated with the solution containing: 180 g/l glycine, 5.5 g/l NaCI and 2.978 g/l disodium ethylenediamine-tetraacetate (EDTA) and additional layer of 0.1 mm thick glass fibre, such as ED-100 from Ahlstrom Filtration, Inc.
Further experimentation with regard to the second screening layer was also performed:
The polycarbonate or polyester membranes in Example 1 or both polycarbonate or polyester membranes in Example 2 were substituted with a polysulfone membrane, UltraBind™ KV-3000 (Gelman Sciences) treated with 10 % (w/v) BSA in water or a polyvinylidene difluoride membrane, 2 um Durapore™ (Millipore) treated with water solutions of 1 % casein or 10 % BSA or 0.1 to 1 % glycerol. The resulting test strips could accommodate approximately 30 ul of blood for analysis.

### EXAMPLE 4.

### MBTH/DMAB Chemistry

The reagent membrane pad for analysis of cholesterol was coated in two steps by dipping and then drying for 15 to 20 min at 65 °C. In the first step the membrane is coated with the organic solvent based solution containing:
571 ml water,
360 ml isopropanol,
33 ml of 30 % CHAPS,
35 ml of 284 mg/ml water solution of sodium cholate,
5 g dimethyl amino benzoic acid (DMAB),
10 g 3-methyl-2-benzothiazolinone hydrazone (MBTH-HCl).

The membrane is then dried at 56 °C for 15 to 20 minutes and than coated in an aqueous solution containing:
308 ml water,
100 ml of 1 M MES buffer, pH 6.0,
80 ml of 500 mg/ml sucrose in water,
83 ml of 6 % (w/v) Gantrez AN-139 Polymer (GAF Chemical Co.),
430 ml of enzyme solution in 0.1 M phosphate buffer, pH 7.0 containing:
200,000 units cholesterol esterase, 100,000 units of lipoprotein lipase,
50,000 units cholesterol oxidase, 170,000 units of horseradish peroxidase.

### EXAMPLE 5.

### Glucose test

The reagent membrane is coated in two steps by dipping and then drying for 15 to 20 min at 65 °C. In the first step the membrane is coated with an organic solvent based solution containing (from U.S. patent #4,935,346):
5 ml water,
5 ml acetonitrile,
80 mg dimethyl amino benzoic acid (DMAB),
40 g 3-methyl-2-benzothiazolinone hydrazone (MBTH-HCl).

The membrane is then dried at 56 °C for 15 to 20 minutes and than coated in an aqueous solution containing:
6 ml water,
10 mg EDTA,
0. 668 g sodium citrate
0. 523 g citric acid
2 ml of 6 % (w/v) Gantrez AN-139 Polymer (GAF Chemical Co.) in water,
3000 units of horseradish peroxidase,
6000 units of glucose oxidase.

## Claims

1. A test strip for the determination of an analyte in a biological fluid comprising:
an initial hydrophilic screening layer;
at least one final hydrophilic screening layer positioned proximate to and adjoining said first layer; and
at least one hydrophilic reagent membrane pad into which is incorporated a dry chemical reagent detection system for such analyte, positioned proximate to and adjoining said final screening layer.

2. The test strip of claim 1 wherein said final screening layer or layers comprise a material selected from the group consisting of polycarbonate, polyester, polysulfonate, and nylon.

3. The test strip of claim 2 wherein said final screening layer or layers comprise polycarbonate.

4. The test strip of claim 3 wherein the pore size of such polycarbonate material ranges from .2 micrometers to 3 micrometers.

5. The test strip of claim 4 wherein said polycarbonate material comprises 2x10⁶ to 1x10⁸ pores per square centimeter.

6. The test strip of claim 5 comprising two final screening layers.

7. The test strip of claim 5 wherein said initial screening layer comprises a material selected from the group consisting of cellulose, polysulfone, polyester, and glass fibers.

8. The test strip of claim 7 wherein said initial screening layer comprises glycine paper.

9. The test strip of claim 1 wherein said reagent membrane pad comprises a dry chemical detection system capable of detecting an analyte selected from the group consisting of glucose, cholesterol, uric acid, protein, and creatinine.

10. The test strip of claim 9 wherein said reagent membrane pad comprises a dry reagent chemical detection system capable of detecting cholesterol.

11. The test strip of claim 7 wherein said reagent membrane comprises a dry reagent chemical detection system capable of detecting cholesterol.

12. The test strip of claim 11 wherein said dry reagent cholesterol detection system comprises cholesterol esterase activity, cholesterol oxidase activity, and a chromogenic detection system.

13. The test strip of claim 12 wherein said cholesterol detection system comprises horseradish peroxidase and a hydrogen peroxide chromogenic detector.

14. The test strip of claim 13 further comprising a chemical agent incorporated into said reagent membrane to prevent excessive wash-out of said chromogenic detection system.

15. The test strip of claim 14 wherein said chemical agent comprises a high molecular weight polymer.

16. The test strip of claim 15 wherein said chemical agent comprises dextran.

17. A dry reagent test strip method for the detection of an analyte in a biological sample comprising:
applying an amount of a liquid biological sample to be tested to the test strip of claim 1;
allowing said sample to travel through said screening layers;
and observing said reagent membrane pad for a color change.

18. A method for the detection of an analyte in a biological sample comprising the steps of:
applying an amount of said biological sample in liquid form to an initial hydrophilic screening layer;
allowing said sample to pass through said initial layer to at least one final screening layer;
allowing said sample to pass through said at least one final screening layer to a reagent membrane pad; and
observing said reagent pad to detect a chromogenic reaction indicative of the presence or absence of analyte.

19. The method of claim 18 wherein said at least one final screening layer is selected from the group consisting of polycarbonate and polyester membranes.

20. The method of claim 19 wherein said analyte is cholesterol.

## Patentansprüche

1. Teststreifen zur Bestimmung eines Analyten in einer biologischen Flüssigkeit, umfassend:
eine hydrophile Anfangssiebschicht;
mindestens eine hydrophile Endsiebschicht, die in der Nähe der ersten Schicht angeordnet ist und an diese angrenzt; und
mindestens ein hydrophiles Reagenz-Membrankissen, dem ein trockenchemisches Reagenznachweissystem für einen derartigen Analyten einverleibt ist und das sich in der Nähe der Endsiebschicht befindet und an diese angrenzt.

2. Teststreifen nach Anspruch 1, wobei die Endsiebschicht oder -schichten ein Material aus der Gruppe Polycarbonat, Polyester, Polysulfonat und Nylon umfassen.

3. Teststreifen nach Anspruch 2, wobei die Endsiebschicht oder -schichten Polycarbonat umfassen.

4. Teststreifen nach Anspruch 3, wobei die Porengröße des Polycarbonatmaterials im Bereich von 0,2 µm bis 3 µm liegt.

5. Teststreifen nach Anspruch 4, wobei das Polycarbonatmaterial 2x10⁶ bis 1x10⁸ Poren pro cm² umfaßt.

6. Teststreifen nach Anspruch 5, umfassend zwei Endsiebschichten.

7. Teststreifen nach Anspruch 5, wobei die Anfangssiebschicht ein Material aus der Gruppe Cellulose, Polysulfon, Polyester und Glasfasern umfaßt.

8. Teststreifen nach Anspruch 7, wobei die Anfangssiebschicht Glycin-Papier umfaßt.

9. Teststreifen nach Anspruch 1, wobei das Reagenzmembrankissen ein trockenchemisches Nachweissystem umfaßt, das zum Nachweis eines Analyten aus der Gruppe Glucose, Cholesterin, Harnsäure, Protein und Creatinin befähigt ist.

10. Teststreifen nach Anspruch 9, wobei das Reagenzmembrankissen ein trockenchemisches Nachweissystem, das zum Nachweis von Cholesterin befähigt ist, umfaßt.

11. Teststreifen nach Anspruch 7, wobei die Reagenzmembran ein trockenchemisches Nachweissystem, das zum Nachweis von Cholesterin befähigt ist, umfaßt.

12. Teststreifen nach Anspruch 11, wobei das Trockenreagenz-Cholesterinnachweissystem Cholesterin-esterase-Aktivität, Cholesterinoxidase-Aktivität und ein chromogenes Nachweissystem umfaßt.

13. Teststreifen nach Anspruch 12, wobei das Cholesterinnachweissystem Meerrettich-peroxidase und einen chromogenen Wasserstoffperoxid-Detektor umfaßt.

14. Teststreifen nach Anspruch 13, ferner umfassend ein der Reagenzmembran einverleibtes chemisches Mittel, um ein übermäßiges Auswaschen des chromogenen Nachweissystems zu verhindern.

15. Teststreifen nach Anspruch 14, wobei das chemische Mittel ein hochmolekulares Polymeres umfaßt.

16. Teststreifen nach Anspruch 15, wobei das chemische Mittel Dextran umfaßt.

17. Trockenreagenz-Teststreifenverfahren zum Nachweis eines Analyten in einer biologischen Probe, umfassend:
das Aufbringen einer Menge einer zu testenden biologischen Probe auf den Teststreifen nach Anspruch 1;
das Wandern der Probe durch die Siebschichten;
und das Beobachten einer Farbänderung des Reagenzmembrankissens.

18. Verfahren zum Nachweis eines Analyten in einer biologischen Probe, umfassend folgende Stufen:
das Aufbringen einer Menge der biologischen Probe in flüssiger Form auf eine hydrophile Anfangssiebschicht;
das Passierenlassen der Probe durch die Anfangsschicht zu mindestens einer Endsiebschicht;
das Passierenlassen der Probe durch die mindestens eine Endsiebschicht zu einem Reagenzmembrankissen; und
das Beobachten des Reagenzkissens zum Nachweis einer chromogenen Reaktion als Anzeichen für die Anwesenheit oder Abwesenheit eines Analyten.

19. Verfahren nach Anspruch 18, wobei die mindestens eine Endsiebschicht aus der Gruppe Polycarbonat- und Polyestermembranen ausgewählt ist.

20. Verfahren nach Anspruch 19, wobei es sich beim Analyten um Cholesterin handelt.

## Revendications

1. Bande de test pour la détermination d'un analyte dans un fluide biologique comprenant :
une couche initiale hydrophile de tamisage;
au moins une couche finale hydrophile de tamisage placée proche de et adjacente à ladite première couche; et
au moins un tampon de membrane de réactifs hydrophile dans lequel est incorporé un système de détection à réactifs chimiques secs pour cet analyte, qui se trouve à proximité de et adjacent à ladite couche finale de tamisage.

2. Bande de test de la revendication 1 où ladite couche finale de tamisage ou les couches comprennent un matériau sélectionné dans le groupe consistant en polycarbonate, polyester, polysulfonate, et nylon.

3. Bande de test de la revendication 2 où ladite couche finale de tamisage ou les couches comprennent un polycarbonate.

4. Bande de test de la revendication 3 où la grandeur des pores de ce matériau de polycarbonate est comprise entre 0,2 micron et 3 microns.

5. Bande de test de la revendication 4 où ledit matériau de polycarbonate comprend 2x10⁶ à 1x10⁸ pores par centimètre carré.

6. Bande de test de la revendication 5 comprenant deux couches finales de tamisage.

7. Bande de test de la revendication 5 où ladite couche initiale de tamisage comprend un matériau sélectionné dans le groupe consistant en cellulose, polysulfone, polyester, et fibres de verre.

8. Bande de test de la revendication 7 où ladite couche initiale de tamisage comprend un papier de glycine.

9. Bande de test de la revendication 1 où ledit tampon de membrane de réactifs comprend un système de détection chimique à sec capable de détecter un analyte sélectionné dans le groupe consistant en glucose, cholestérol, acide urique, protéine et créatinine.

10. Bande de test de la revendication 9 où ledit tampon de membrane de réactifs comprend un système de détection chimique à réactifs secs capable de détecter le cholestérol.

11. Bande de test de la revendication 7 où ladite membrane de réactifs comprend un système de détection chimique de réactifs secs capable de détecter le cholestérol.

12. Bande de test de la revendication 11 où ledit système de détection du cholestérol avec réactifs secs comprend l'activité de cholestérol estérase, l'activité de cholestérol oxydase, et un système de détection chromogène.

13. Bande de test de la revendication 12 où ledit système de détection du cholestérol comprend la péroxydase de raifort et un détecteur chromogène du péroxyde d'hydrogène.

14. Bande de test de la revendication 13 comprenant de plus un agent chimique incorporé dans ladite membrane de réactifs pour empêcher un délavage excessif dudit système de détection chromogène.

15. Bande de test de la revendication 14 où ledit agent chimique comprend un polymère de fort poids moléculaire.

16. Bande de test de la revendication 15 où ledit agent chimique comprend du dextrane.

17. Méthode à bande de test à réactifs secs pour la détection d'un analyte dans un échantillon biologique consistant à :
appliquer une quantité d'un échantillon biologique liquide à tester à la bande de test de la revendication 1;
laisser l'échantillon se déplacer à travers lesdites couches de tamisage;
et observer ledit tampon de membrane de réactifs pour un changement de couleur.

18. Méthode pour la détection d'un analyte dans un échantillon biologique, comprenant les étapes de :
appliquer une quantité dudit échantillon biologique sous forme liquide à une couche initiale hydrophile de tamisage;
laisser ledit échantillon passer à travers ladite couche initiale jusqu'à au moins une couche finale de tamisage;
laisser ledit échantillon passer à travers ladite au moins une couche finale de tamisage vers un tampon de membrane de réactifs; et
observer ledit tampon de réactifs pour détecter une réaction chromogène indiquant la présence ou l'absence d'un analyte.

19. Méthode de la revendication 18 où ladite au moins une couche finale de tamisage est sélectionnée dans le groupe consistant en membranes de polycarbonate et de polyester.

20. Méthode de la revendication 19 où ledit analyte est du cholestérol.
